(19) Europäisches Patentamt
European Patent Office
Office européen des brevets

(11) Veröffentlichungsnummer: **0 304 696 B1**

(12) **EUROPÄISCHE PATENTSCHRIFT**

(45) Veröffentlichungstag der Patentschrift: **29.04.92**

(51) Int. Cl.5: **C07D 307/08**, C07C 29/136, //B01J23/70,B01J27/185, B01J27/18

(21) Anmeldenummer: **88112672.6**

(22) Anmeldetag: **04.08.88**

# (54) Verfahren zur Herstellung von 1,4-Butandiol und/oder Tetrahydrofuran.

(30) Priorität: **08.08.87 DE 3726509**
**08.08.87 DE 3726510**

(43) Veröffentlichungstag der Anmeldung:
**01.03.89 Patentblatt 89/09**

(45) Bekanntmachung des Hinweises auf die Patenterteilung:
**29.04.92 Patentblatt 92/18**

(84) Benannte Vertragsstaaten:
**BE CH DE FR GB IT LI NL**

(56) Entgegenhaltungen:
**DE-A- 1 939 882** **DE-A- 2 133 768**
**DE-B- 1 285 992** **DE-B- 2 339 344**
**US-A- 2 772 292** **US-A- 2 772 293**

**CHEMICAL ABSTRACTS, Band 83, Nr. 5, 4. August 1975, Seite 86, Abstract 43176t; & JP - A - 75 01038**

**CHEMICAL ABSTRACTS, Band 81, Nr. 17, 28. Oktober 1974, Seite 506, Abstract 105257g; & JP - A - 74 09464**

(73) Patentinhaber: **BASF Aktiengesellschaft**
**Carl-Bosch-Strasse 38**
**W-6700 Ludwigshafen(DE)**

(72) Erfinder: **Fischer, Rolf, Dr.**
**Bergstrasse 98**
**W-6900 Heidelberg(DE)**
Erfinder: **Gosch, Hans-Juergen, Dr.**
**Seebacher Strasse 37**
**W-6702 Bad Duerkheim(DE)**
Erfinder: **Harder, Wolfgang, Dr.**
**Bergwaldstrasse 16**
**W-6940 Weinheim(DE)**
Erfinder: **Malsch, Klaus-Dieter, Dr.**
**Selligstrasse 80**
**W-6707 Schifferstadt(DE)**
Erfinder: **Eggersdorfer, Manfred, Dr.**
**Hannongstrasse 18**
**W-6710 Frankenthal(DE)**
Erfinder: **Franz, Lothar, Dr.**
**Sternstrasse 197**
**W-6700 Ludwigshafen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 304 696 B1

**CHEMICAL ABSTRACTS, Band 80, Nr. 17, 29. Aprill 1974, Seite 392, Abstract 95707z; & JP - A - 73 30634**

Erfinder: **Zimmermann, Horst, Dr.**
**Ringstrasse 36**
**W-6900 Heidelberg(DE)**
Erfinder: **Brenner, Karl**
**Riedsaumstrasse 40**
**W-6700 Ludwigshafen(DE)**
Erfinder: **Halbritter, Klaus, Dr.**
**Schwarzwaldstrasse 19**
**W-6800 Mannheim 1(DE)**
Erfinder: **Sauer, Wolfgang, Dr.**
**Rosbergstraat 18**
**B-3078 Everberg(BE)**
Erfinder: **Scheiper, Hans-Juergen**
**Pfalzring 46**
**W-6704 Mutterstadt(DE)**

## Beschreibung

Diese Erfindung betrifft ein Verfahren zur Herstellung von 1,4-Butandiol und/oder Tetrahydrofuran durch katalytische Hydrierung von Maleinsäureanhydrid, Bernsteinsäureanhydrid, Maleinsäure, Bernsteinsäure, Fumarsäure oder den Alkylestern dieser Säuren.

Im folgenden Text werden die folgenden Kurzbezeichnungen verwendet:
MS = Maleinsäure, BS = Bernsteinsäure, MSA = Maleinsäureanhydrid, BSA = Bernsteinsäureanhydrid, THF = Tetrahydrofuran, GBL = $\gamma$-Butyrolacton, BD = 1,4-Butandiol, FS = Fumarsäure.

THF, BD und GBL sind wertvolle Zwischenprodukte. So werden THF und BD z.B. für die Herstellung von Polyurethanen, Polybutylenterephthalat oder Polytetrahydrofuran und GBL für die Herstellung von Pyrrolidon verwendet. THF und GBL finden auch als Lösungsmittel Verwendung.

Bekanntlich lassen sich GBL, THF und BD durch katalytische Hydrierung von MSA herstellen. Da diese Hydrierung entsprechend dem in der US-PS 4,155,919 angegebenen Reaktionsschema verläuft, hat man sich bemüht, die Hydrierung durch bestimmte Verfahrensmaßnahmen so zu steuern, daß zumindest eines dieser Reaktionsprodukte bevorzugt erhalten wird. Die Produktverteilung sieht bei den bekannten Verfahren meist so aus, daß entweder GBL oder BD und THF erhalten wird.

GBL erhält man z.B. nach dem in der US-PS 4,096,156 beschriebenen Verfahren, bei dem man MS, MSA, BSA und/oder FS in Gegenwart von Edelmetallkatalysatoren hydriert. Nachteilig an diesem Verfahren ist, daß die sehr teuren Edelmetalle benötigt werden und der Einsatz der komplexen Gemische der Edelmetalle mit einer sehr arbeitsaufwendigen Rückgewinnung der Edelmetalle verbunden ist.

Aus der DE-PS 21 33 768 ist bekannt, daß sich Katalysatoren, die neben Cobalt, Rhenium und/oder Molybdän enthalten, für die Hydrierung von BSA oder MSA zur Gewinnung von THF und GBL eignen. Diese Katalysatoren haben sich als sehr empfindlich gegen Säuren und andere Verunreinigungen erwiesen.

In der US-PS 2 772 292 wird ein einstufiges Verfahren beschrieben, bei dem man MSA in Gegenwart von Raney-Cobalt hydriert. Dabei erhält man nach Beispiel 1 bei 275 °C und etwa 800 bar BD in 64 %iger Ausbeute; außerdem werden 11 % THF gebildet. Nach den Angaben der DE-OS 2 133 768 hydriert man MSA an einem Katalysator, der neben Cobalt Rhenium und/oder Molybdän enthält. Nach Beispiel 3 läßt sich MSA mit Hilfe eines Katalysators aus Cobalt und Rhenium (Atomverhältnis 1:0,03) auf $SiO_2/Al_2O_3$ als Träger zu einem Gemisch hydrieren, das zu 6,9 % aus BD, zu 22,3 % aus THF und zu 63,7 % aus GBL besteht.

Man hat MSA auch schon in Gegenwart von aliphatischen Alkoholen zu BD hydriert. Dieses in der DE-PS 2 845 905 beschriebene einstufige Verfahren hat den Nachteil, daß man einen Kupferchromit-Katalysator benötigt, bei dessen Herstellung toxische Ausgangsstoffe verarbeitet werden müssen. Außerdem sind die mit derartigen Katalysatoren erzielten Standzeiten für eine großtechnische Produktion nicht befriedigend.

Es war deshalb nach einem Verfahren zur Herstellung von BD und/oder THF aus den genannten Ausgangsverbindungen zu suchen, das diese katalysatorbedingten Nachteile nicht aufweist. Der Katalysator sollte insbesondere billige Nichtedelmetalle enthalten, eine lange Standzeit und eine große Stabilität gegenüber Korrosion aufweisen sowie hohe Ausbeuten an BD, THF und an dem als Zwischenstufe gebildeten GBL ermöglichen. Es war außerdem wünschenswert, diese drei Produkte in variablen Mengenverhältnissen herstellen zu können.

Nach dem erfindungsgemäßen Verfahren, das diese Forderungen weitgehend erfüllt, stellt man BD und/oder THF durch katalytische Hydrierung von Verbindungen der allgemeinen Formel

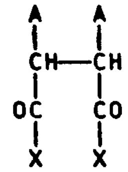

in der A ein Wasserstoffatom oder beide A zusammen eine Einfachbindung, X eine HO- oder RO-Gruppe, R einen Alkylrest mit 1 bis 6 C-Atomen, oder beide X zusammen das Ringglied -O- bedeuten, bei Temperaturen von 100 bis 350 °C und Drücken von 50 bis 350 bar dadurch her, daß man einen Katalysator verwendet, der als katalytisch aktives Metall Cobalt und mindestens eines der Elemente Kupfer und Phosphor sowie gegebenenfalls noch eines oder mehrere der Elemente Mangan, Molybdän und Natrium enthält.

In der DE-AS 12 85 992 ist ein Verfahren zur Hydrierung von Alkinolen und Alkindiolen mittels eines Katalysators beschrieben, der Cobalt, Kupfer und Zusätze von Mangan und/oder Chrom enthält.

Aus der DE-AS 23 39 344 ist ein Katalysator bekannt, der Cobalt, Kupfer und Chrom enthält und der Gewinnung von THF aus GB dient.

Ausgangsstoffe der allgemeinen Formel sind MS, MSA, BS, BSA, FS oder Alkylester der MS oder BS, von denen MS, MSA oder Gemische aus MS und MSA bevorzugt sind. Diese Ausgangsstoffe werden z.B. in Form einer Schmelze oder als Lösungen der Hydrierung unterworfen. Als Lösungen verwendet man z.B. 5 bis 60 gew.-%ige Lösungen von MSA oder MS in Lösungsmitteln, wie Wasser, GBL oder Ether, wie Dioxan, Tetrahydropyran, THF und Diethylether. Beispielsweise verwendet man eine 10 bis 60, vorzugsweise 20 bis 40-gewichtsprozentige Lösung von MSA in THF oder eine 5 bis 40, vorzugsweise 25 bis 40-gewichtsprozentige Lösung von MS in Wasser.

Bei diesen Verfahrensvarianten hydriert man im Temperaturbereich von 150 bis 350 ° C, vorzugweise bei 170 bis 250 ° C und bei Drücken von 50 bis 350 bar, vorzugweise 250 bis 300 bar. Die Hydrierung wird diskontinuierlich, vorzugsweise aber kontinuierlich durchgeführt.

Die erfindungsgemäß zu verwendenden Katalysatoren enthalten Cobalt und mindestens eines der Elemente Kupfer und Phosphor. Bevorzugt enthalten die Katalysatoren neben Cobalt mindestens zwei der Elemente Kupfer, Phosphor und Molybdän. Von besonderem technischem Interesse sind solche Katalysatoren, die neben Cobalt mindestens drei der Elemente Kupfer, Phosphor, Molybdän und Mangan enthalten. Dabei können die Metalle in metallischer Form oder in Form ihrer Oxide eingesetzt werden. Phosphor liegt praktisch in Form von Phosphorsäure vor.

Geeignete Katalysatoren sind z.B. solche, deren katalytisch aktive Masse mindestens zu 40 Gew.% aus Cobalt besteht und als weitere aktive Bestandteile bis zu 20 Gew.%, z.B. 5 bis 18 Gew.% Kupfer, bis zu 8 Gew.%, z.B. 0,1 bis 7 Gew.% Phosphorsäure und bis zu 10 Gew.%, z.B. 0,1 bis 8 Gew.% Molybdän enthält. Beispielsweise seien Katalysatoren genannt, deren katalytisch aktive Masse zu 40 bis 60 Gew.% aus Cobalt, 13 bis 17 Gew.% aus Kupfer, 0,5 bis 5 Gew.% aus Molybdän (berechnet als $MoO_3$), 0 bis 8 Gew.% aus Mangan und 0,1 bis 5 Gew.% aus Phosphor (berechnet als $H_3PO_4$) besteht. Die Katalysatoren können, z.B. bis zu 10 Gew.% an sich übliche Inertmaterialien, wie Siliziumdioxid, Aluminiumoxid, Zeolithe, Titanoxid, Thoriumoxid, Magnesiumoxid, Bims, Rutil, Zirkonoxid oder Kohlenstoff enthalten. Der Katalysator, der die metallischen Bestandteile üblicherweise in Form ihrer Oxide enthält, wird in der Regel vor seinem Einsatz und zweckmäßigerweise in der Hydrierapparatur durch eine Wasserstoffbehandlung aktiviert, wobei die Oxide größtenteils zu den Metallen reduziert werden.

Die Standzeit des Katalysators beträgt ohne Aktivitätsabfall mehr als 7 Monate. Die Raum-Zeit-Ausbeute liegt bei der Schmelzfahrweise zwischen 0,2 und 0,9 kg/l . h und bei der Lösungsmittelfahrweise zwischen 0,2 und 0,8 kg/l . h.

Der zur Hydrierung eingesetzte Wasserstoff wird im allgemeinen in größerem stöchiometrischen Überschuß verwendet. Er kann als Kreisgas in die Reaktion zurückgeführt werden. Der Wasserstoff kommt im allgemeinen technisch rein zum Einsatz. Beimengen von Inertgasen, z.B. Stickstoff stören jedoch den Reaktionsablauf nicht.

Beim kontinuierlichen Betrieb kann sowohl in Sumpf- als auch in Rieselfahrweise gearbeitet werden. Hydriert man diskontinuierlich, so verfährt man z.B. so, daß man eine Lösung von MSA in THF zusammen mit dem Katalysator in einen Hochdruckautoklaven gibt, Wasserstoff aufpreßt und das Reaktionsgemisch erhitzt. Nach Ende der Reaktion wird abgekühlt, der Katalysator abgetrennt und das Reaktionsgemisch zur Isolierung von THF, GBL und BD fraktioniert destilliert. Man kann aber auch, falls gewünscht, das entstandene GBL in einer Nachhydrierung zu BD und THF umsetzen und das BD auf bekannte Weise zyklisieren.

Das erfindungsgemäße Verfahren erlaubt es, den mengenmäßigen Anteil der Verfahrensprodukte im Reaktionsgemisch im Bereich von 20 bis 100 Gew.% THF, 0 bis 55 Gew.% GBL und 0 bis 60 Gew.% BD zu variieren. So erhält man Reaktionsgemische mit hohem THF-Anteil, wenn man bei hohen Temperaturen und/oder niedriger Katalysatorbelastung arbeitet, während die Bildung von GBL durch niedrigere Temperaturen und/oder hohe Katalysatorbelastung begünstigt wird.

Beispielsweise wird bei einer Reaktionstemperatur von 260 ° C und einer Katalysatorbelastung von 0,4 kg/l . h ein Reaktionsaustrag aus 50,8 Gew.% THF, 38,8 Gew.% BD und 4,6 Gew.% GBL erhalten, während sich die Zusammensetzung des Produktstromes zu 29,5 Gew.% THF, 29,5 Gew.% BD und 34,1 Gew.% GBL verschiebt, wenn bei einer Temperatur von 210 ° C und gleicher Katalysatorbelastung gearbeitet wird. Verändert man die Kontaktbelastung auf 0,1 kg/l . h und stellt eine Temperatur von 220 ° C ein, so erhält man ein Gemisch aus 23 Gew.% THF, 66 Gew.% BD und 2 Gew.% GBL. Reduziert man die Temperatur bei derselben Kontaktbelastung auf 170 ° C, so wird folgende Zusammensetzung erhalten: 5 Gew.% THF, 36 Gew.% BD und 58 Gew.% GBL.

Nach dem neuen Verfahren erhält man die Verfahrensprodukte bei quantitativer Umsetzung der Ausgangsstoffe und bei hohen Raum-Zeit-Ausbeuten in hohen Ausbeuten. Überraschenderweise werden Korrosions- und Desaktivierungserscheinungen am Katalysator weder beim Einsatz von MSA-Schmelzen noch bei Verwendung wäßriger MS-Lösungen beobachtet. Außerdem ist die Nebenproduktebildung sehr gering. So wird auch die üblicherweise bei Hydrierungen von MSA im Temperaturbereich von > 300 ° C festgestellte Bildung hochmolekularer, pechähnlicher Produkte nicht beobachtet.

Nach einer weiteren vorteilhaften Ausführungsform der Erfindung hydriert man in Gegenwart eines aliphatischen Alkohols und eines Katalysators, der Cobalt und mindestens eines der Elemente Kupfer, Phosphor oder Mangan enthält. Im Vergleich zu den bekannten MSA-Hydrierungen an Cobaltkatalysatoren erhält man hierbei überraschenderweise BD mit Selektivitäten von über 80 % und Gesamtselektivitäten an BD + THF + GBL von 98 %. Neben BD können je nach Reaktionsbedingungen und Katalysator wechselnde Mengen der Wertprodukte THF und GBL erhalten werden.

Man kann auch das entstandene GBL in einer Nachhydrierung zu BD und THF umsetzen.

Da sich schon beim Lösen von MSA in Alkoholen bei niedrigen Temperaturen der jeweiligen Maleinsäuremonoester bildet, läßt sich die Umsetzung von MSA mit Alkoholen und Wasserstoff durch die Teilgleichungen a) und b) beschreiben. Der eingesetzte Alkohol wird bei der Hydrierung zurückgebildet. Daher wird nach der Bruttogleichung (c) MSA mit 5 Molen Wasserstoff zu 1 Mol BD und 1 Mol Wasser umgesetzt:

a) 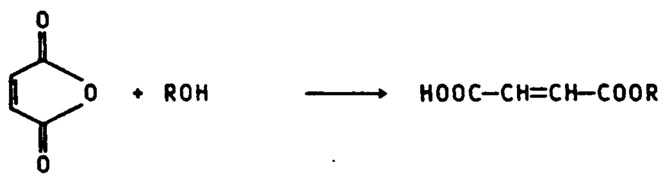

b) $HOOC{-}CH{=}CH{-}COOR + 5\ H_2 \longrightarrow HO{-}CH_2{-}CH_2{-}CH_2{-}CH_2{-}OH + H_2O + ROH$

c) 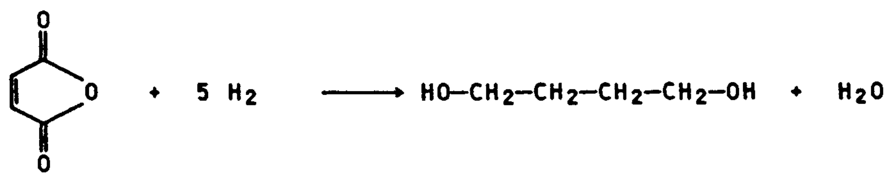

MSA kann in fester, flüssiger oder gasförmiger Form verwendet werden. Für die Hydrierung wird es in dem jeweiligen Alkohol gelöst. Dies ist schon bei Raumtemperatur oder z.B. bei Temperaturen von 30 bis 70 ° C leicht zu erreichen. Besonders vorteilhaft ist es, wenn man das bei der katalytischen Oxidation von n-Butan oder Butenen entstehende, MSA und Wasser enthaltene Gasgemisch, z.B. nach dem Verfahren der EP-PS 149 144, mit Alkoholen zur Absorption des Maleinsäureanhydrids behandelt. Die Abtrennung des im MSA enthaltenen Wassers ist in diesem Fall besonders einfach durchführbar. Es ist aber auch möglich, das Absorbat ohne Abtrennung des Wasser zu hydrieren.

Man kann anstelle von MSA auch von BSA ausgehen. In diesem Fall arbeitet man bei Temperaturen von 30 bis 100 ° C. Außerdem ist es möglich, die Halb-oder Diester der Maleinsäure oder Bernsteinsäure, wie z.B. die Alkylester dieser Säuren, in die Hydrierung einzusetzen. Dabei leiten sich die Alkylester z.B. von Alkanolen mit 1 bis 6 C-Atomen ab, wie von Methanol, Ethanol, i-Propanol, n-Propanol, Hexanole, n-Butanol, i-Butanol. Man hydriert bei dieser Verfahrensvariante in Gegenwart eines Alkohols. Als Alkohole verwendet man z.B. aliphatische Alkohole, insbesondere einwertige Alkanole mit 1 bis 6 C-Atomen wie die obengenannten Alkanole. Besonders bevorzugt sind die Butanole, da sie eine einfache Abtrennung des bei der Hydrierung entstehenden Wassers durch Azeotropdestillation ermöglichen. Es sind aber auch andere ein- oder mehrwertige primäre, sekundäre oder tertiäre aliphatische oder cycloaliphatische Alkohole geeignet. Diese bieten aber wegen ihres im allgemeinen höheren Siedepunktes und wegen der erhöhten Zahl von Reaktionsmöglichkeiten keine Vorteile. Das Molverhältnis von MSA (einschließlich BSA, der Halb- und/oder Diester der Malein- oder Bernsteinsäure) zu den Alkoholen beträgt im allgemeinen 1:0,1 bis 1:30, vorzugsweise 1:0,5 bis 1:20, insbesondere 1:1 bis 1:10.

Die Hydrierung in Gegenwart der Alkohole wird im allgemeinen bei Temperaturen von 100 bis 350 ° C, insbesondere 150 bis 300 ° C, durchgeführt. Man arbeitet bei Drücken von 50 bis 350 bar, insbesondere 100 bis 300 bar. Die Hydrierung wird diskontinuierlich, insbesondere aber kontinuierlich, durchgeführt. Man

arbeitet im Sumpf-, insbesondere aber Rieselfahrweise, wobei der Katalysator fest angeordnet ist. Es ist aber auch möglich, einen suspendierten Katalysator einzusetzen. Um die Hydrierwärme ableiten zu können, kann man sowohl Wasserstoff als auch einen Teil des Hydrieraustrags in die Hydrierstufe zurückleiten.

Als Reaktoren können z.B. Rohrreaktoren oder Rohrbündelreaktoren verwendet werden, wobei die Reaktionswärme durch Außenkühlung oder im Fall von Rohrreaktoren durch Innenkühlung abgeführt werden kann. Als Reaktormaterialien sind z.B. normale Edelstähle, z.B. ®V-Stähle geeignet.

Bei dieser Arbeitsweise verwendet man Katalysatoren, die Cobalt und mindestens eines der Elemente Mangan, Kupfer und Phosphor enthalten. Bevorzugt enthalten die Katalysatoren neben Cobalt mindestens zwei der Elemente Mangan, Kupfer, Phosphor und Molybdän. Von besonderem technischem Interesse sind solche Katalysatoren, die neben Cobalt mindestens drei der Elemente Mangan, Kupfer, Phosphor, Molybdän und Natrium enthalten. Dabei können die Metalle in metallischer Form oder in Form ihrer Oxide nach Reduktion, z.B. mit Wasserstoff, eingesetzt werden. Phosphor liegt praktisch in Form von Phosphorsäure vor.

Geeignete Katalysatoren sind z.B. solche, deren katalytisch aktive Masse mindestens zu 40 Gew.% aus Cobalt besteht und als weitere aktive Bestandteile bis zu 10 Gew.%, z.B. 3 bis 7 Gew.% Mangan, bis zu 20 Gew.%, z.B. 0,1 bis 5 Gew.% Phosphorsäure und bis zu 1 Gew.%, z.B. 0,01 bis 0,5 Gew.% Natrium enthält. Besonders gute Ergebnisse werden z.B. auch mit solchen Katalysatoren dieser Art erzielt, deren katalytisch aktive Masse als weitere aktive Bestandteile bis zu 30 Gew.%, z.B. 12 bis 18 Gew.% Kupfer und bis zu 5 Gew.%, z.B. 1 bis 4 Gew.% Molybdän enthalten.

Die Katalysatoren werden als Trägerkatalysatoren oder vorzugsweise als Substanzkatalysatoren eingesetzt. Trägerkatalysatoren werden zweckmäßigerweise durch ein- oder mehrmaliges Imprägnieren des Trägermaterials mit einer wäßrigen Lösung der Metallsalze hergestellt. Nach dem Imprägnieren und Trocknen wird die Masse erhitzt, wobei die Metallsalze in die Metalloxide übergehen. Vor ihrem Einsatz werden die Katalysatoren mit Wasserstoff behandelt, wobei die Oxide größtenteils zu den Metallen reduziert werden.

Als Katalysatorträger sind z.B. Siliciumdioxid, Aluminiumoxid, Titandioxid, Aktivkohle, Silikate oder Zeolithe geeignet. Wenn nötig, können bei der Katalysatorherstellung Bindemittel verwendet werden.

Die in den Beispielen genannten Prozente sind Gewichtsprozente.

Beispiel 1

Ein Rieselreaktor mit der Länge von 3 m und einem Innendurchmesser von 16 mm wurde mit Katalysatorsträngen gefüllt, die eine Länge von 6 mm und einen Durchmesser von 3 mm hatten. Der Katalysator enthielt die folgenden katalytisch aktiven Bestandteile: 3 % $H_3PO_4$, 64,2 % CoO (≙ 50,5 % Co), 18,5 % CuO (≙ 14,8 % Cu), 6,7 % $Mn_3O_4$ (≙ 4,8 % Mn), 3,7 % $MoO_3$. Am Kopfende des Reaktors wurde eine 40%ige wäßrige Maleinsäurelösung eingeleitet. Wasserstoff wurde ebenfalls am Kopfende mit einem Druck von 300 bar eingeleitet. Bei einem stündlichen Zulauf von 200 g der Maleinsäurelösung wurde ein Umlauf von 9 l/h eingestellt. Es wurden drei Versuche bei unterschiedlichen Temperaturen durchgeführt. In der nachstehenden Tabelle sind die Ausbeuten bei diesen Versuchen (bezogen auf die eingesetzte Maleinsäure) tabellarisch zusammengesetzt.

| t/°C | THF | GBL | BD | Propanol | Butanol | Rückstand |
|---|---|---|---|---|---|---|
| 260 | 50,8 | 4,6 | 38,8 | 0,9 | 2,6 | 1,8 |
| 230 | 45,4 | 18,6 | 30,3 | 0,1 | 2,3 | 2,5 |
| 210 | 29,5 | 34,1 | 29,6 | 0,1 | 1,3 | 1,2 |

Beispiel 2

Entsprechend Beispiel 1 wurde ein Katalysator mit folgender Zusammensetzung eingesetzt: 3 % $H_3PO_4$, 64,2 % CoO (≙ 50,5 % Co), 18,5 % CuO (≙ 14,8 % Cu) und 3,7 % $MoO_3$. Die dabei erhaltenen Ausbeuten sind der folgenden Tabelle zu entnehmen.

| t/°C | THF | GBL | BD | Propanol | Butanol | Rückstand |
|---|---|---|---|---|---|---|
| 280 | 33,1 | 17,3 | 31,0 | 4,0 | 13,7 | 1,3 |
| 270 | 38,9 | 16,0 | 27,0 | 3,4 | 13,5 | 1,4 |
| 260 | 42,1 | 15,8 | 25,3 | 3,0 | 12,3 | 1,5 |

Beispiel 3

Entsprechend Beispiel 1 wurde ein Katalysator mit folgender Zusammensetzung eingesetzt: 8,5 % $H_3PO_4$, 62,0 % CoO (≙ 48,7 % Co), 17,8 % CuO (≙ 14,2 % Cu), 6,4 % $Mn_3O_4$ (≙ 4,5 % Mn), 3,6 % $MoO_3$. Die dabei erhaltenen Ausbeuten sind der folgenden Tabelle zu entnehmen.

| t/°C | THF | BL | BD | Propanol | Butanol | Rückstand |
|---|---|---|---|---|---|---|
| 260 | 61,5 | 0,2 | 9,7 | 10,2 | 16,8 | 1,5 |
| 240 | 56,7 | 0,6 | 26,7 | 5,0 | 9,6 | 1,4 |
| 210 | 21,2 | 10,5 | 63,3 | 0,9 | 2,1 | 2,0 |

Beispiel 4

Ein für die Sumpffahrweise geeigneter Reaktor mit einer Länge von 2 m und einem Durchmesser von 41 mm wurde mit Katalysatorsträngen der Länge von 7 mm und dem Durchmesser von 4 mm gefüllt. Der Katalysator hatte die im Beispiel 1 genannte Zusammensetzung. In den Reaktor wurde von unten eine Schmelze von MSA eingeleitet. Wasserstoff wurde von unten mit einem Druck von 300 bar in den Reaktor eingeleitet. Bei einem stündlichen Zulauf von 0,7 1 der Schmelze wurde ein Umlauf von 20 l/h eingestellt. Bei einer Reaktortemperatur von 265°C wurden die folgenden Ausbeuten erhalten: 33 % BD, 46 % THF, 11 % GBL, 4 % Butanol, 1 % Propanol (Angaben in mol-%).

Beispiel 5

Die Hydrierung wurde wie im Beispiel 4 beschrieben durchgeführt, wobei jedoch ein Zulauf von 0,3 l/h, ein Umlauf von 8 l/h und eine Reaktortemperatur von 245°C eingestellt wurden. Dabei wurden die folgenden Ausbeuten erhalten: 46 % BD, 37 % THF, 7 % GBL, 4 % Butanol, 1 % Propanol (Angaben in mol-%).

Beispiel 6

In einem Rieselreaktor mit einer Länge von 10 m und einem Durchmesser von 41 mm, der mit dem in Beispiel 4 verwendeten Katalysatorsträngen gefüllt war, wurde entsprechend Beispiel 4 eine Schmelze von MSA und Wasserstoff eingeleitet. Bei einem Zulauf von 7 l Schmelze/h wurde ein Umlauf von 40 l/h eingestellt. Die Reaktortemperatur betrug 305°C . Dabei wurden die folgenden Ausbeuten erhalten: 15 % BD, 55 % THF, 25 % GBL, 1,5 % Butanol, 0,5 % Propanol (Angaben in mol-%).

Beispiel 7

Die Hydrierung wurde wie in Beispiel 6 beschrieben durchgeführt, wobei jedoch die Temperatur am Reaktoreingang 240°C und am Reaktorausgang 280°C betrug. Dabei wurden die folgenden Ausbeuten erhalten: 15 % BD, 55 % THF, 25 % GBL, 2 % Butanol und Propanol.

In einem weiteren Versuch wurde das Reaktionsgemisch aus dem Reaktorausgang in einen Nachreaktor geleitet, der die gleiche Dimensionierung wie der erste Reaktor aufwies und wie der erste Reaktor mit dem Katalysator beschickt war. Die Nachhydrierung wurde bei einem Wasserstoffdruck von 300 bar ohne Kreisflüssigkeitsfahrweise vorgenommen. Dabei wurde am Reaktorausgang eine Temperatur von 260°C eingestellt. Es wurden die folgenden Ausbeuten erhalten: 93 % BD und THF, 1,5 % GBL, 2,5 % Butanol und 0,4 % Propanol.

Beispiel 8

In das Reaktorrohr (1) der in der Abbildung schematisch dargestellten Kreislaufapparatur mit der Länge 2000 mm und dem Innendurchmesser 16 mm wurden 612 g eines Katalysators eingebaut, der die folgenden katalytisch aktiven Bestandteile enthielt: 63,4 % CoO, 18,1 % CuO, 6,8 % $Mn_3O_4$, 3,1 % $MoO_3$, 0,15 % $Na_2O$ und 3,3 % $H_3PO_4$ (4-mm Stränge). Der Gehalt des Katalysators an den aktiven Metallen beträgt somit 50,1 % Co, 14,5 % Cu, 4,9 Gew.% Mn, 2,1 % Mo und 0,1 % Na. Die Apparatur wurde unter Zuleitung von Stickstoff auf 100 °C aufgeheizt. Dann wurde über die Zuleitung (2) ein Gemisch aus Stickstoff und Wasserstoff (1 bis 25 Vol.% $H_2$) durch die Apparatur geleitet. Die Temperatur wurde mit einer Heizrate von 20 °C/h auf 220 °C gebracht und dann 40 Stunden bei dieser Temperatur belassen. Nach der Katalysatorreduktion wurde ein Gemisch aus MSA und n-Butanol im Molverhältnis 1:2,5 (50 g/h) mit der Zulaufpumpe (3) aus dem Behälter (4) zusammen mit Wasserstoff aus der Zuleitung (2) in den Hydrierkreislauf gepumpt. Mit Hilfe des Wärmetauschers (5) wurde die Temperatur auf 200 bis 230 °C eingestellt.

Über die Standhaltung im Abscheider (6) wurde eine dem Zulauf entsprechende Produktmenge kontinuierlich ausgeschleust (7). Die Produktrückführung (8) erfolgte mit der Kreislaufpumpe (9). Das Abgas wurde über die Ableitung (10) abgeleitet. Die Hydrierung des Gemischs wurde während 3500 Stunden Betriebszeit unter den folgenden Bedingungen durchgeführt:
Druck: 200 bis 250 bar
Temperatur: 200 bis 250 °C
Abgasmenge: 50 bis 100 l/h
Flüssigkeitskreislauf: 15 l/h
Querschnittsbelastung: > 80 $m^3/m^2 \times h$
Nach 3500 Stunden konnte kein merkliches Ablassen der Katalysatoraktivität festgestellt werden.

Nach quantitativer GC-Analyse wurden bei folgenden Bedingungen - Temperatur 230 °C, Druck 200 bar und Abgas 100 l/h - folgende Ergebnisse erzielt:
Umsatz zu BD, GBL und THF: 98 %
Selektivität zu BD: 82 %
Selektivität zu GBL: 5 %
Selektivität zu THF: 11 %

Beispiel 9

In einem 1,5 l-Rührautoklaven wurden 100 ml (186 g) eines Katalysators der Zusammensetzung 92,3 % CoO, 5,3 % $Mn_3O_4$, 2,8 % $H_3PO_4$ und 0,3 % $Na_2O$ eingebaut. Der Gehalt des Katalysators an den aktiven Metallen beträgt somit: 72,5 % Co, 3,8 % Mn und 0,2 % Na. Unter Überleiten von Stickstoff (ca. 300 Nl/Std) wurde auf 220 °C aufgeheizt. Anschließend wurde dem Stickstoff zunächst 1 Vol.% Wasserstoff zugegeben und die Wasserstoffmenge langsam auf 7 Vol.% erhöht. Man hielt die Temperatur 24 Std. auf 220 °C und ließ danach wieder auf Raumtemperatur abkühlen. Nun wurde der Autoklav mit 880 g eines Gemisches aus MSA und n-Butanol im Molverhältnis 1:2,5 beschickt. Dann wurde die Mischung unter folgenden Reaktionsbedingungen hydriert:
Temperatur: 200 °C
$H_2$-Druck: 200 bar
Turbinendrehzahl: 2 000 U/min
Nach 21 bzw. 46 Stunden wurden dem Autoklaven Flüssigkeitsproben entnommen und mittels Gas- und Flüssigkeitschromatographie analysiert. Es ergaben sich folgende Konzentrationen (Mol.%) im Austrag:

| | nach 21 Std. | nach 46 Std. |
|---|---|---|
| Bernsteinsäuremonobutylester | 3 % | - |
| Bernsteinsäuredibutylester | 9 % | 1 |
| BD | 23 % | 85 % |
| GBL | 36 % | 3 % |
| THF | 3 % | 3 % |

Der Umsatz zu den Wertprodukten BD, GBL und THF betrug nach 21 Std. 62 % und nach 46 Std. 91 %.

Beispiel 10

In einen Rohrreaktor mit einer Länge von 500 mm und einem Innendurchmesser von 15 mm wurden 104 g Katalysators mit der gleichen Zusammensetzung wie in Beispiel 8 eingebaut. Der Reaktor wurde unter Zuleitung von Stickstoff auf 290 °C aufgeheizt. Dann wurde innerhalb von 6 Stunden der Stickstoff gleichmäßig durch Wasserstoff ersetzt und anschließend noch 48 Stunden bei 300 bis 310 °C reiner Wasserstoff durch den Reaktor geleitet. Nach der Katalysatoraktivierung wurde der Reaktor auf 250 °C thermostatisiert. Anschließend wurde ein Gemisch aus MSA und n-Butanol im Molverhältnis 1:2,5 zusammen mit Wasserstoff in den Reaktor gepumpt. Über ein Druckhalteventil mit angeschlossenem Kühler wurde das Produkt aus dem Reaktor ausgeschleust. Nach quantitativer GC-Analyse wurde bei 250 °C und bei 200 bar mit 60 Nl/h Wasserstoff und einer Verweilzeit von 90 Minuten folgendes Ergenbnis erzielt:
62 Mol.% BD, 17 Mol.% THF, 11 Mol.% GBL. Die Ausbeute an Wertprodukten betrug somit 90 %, die Selektivität unter Berücksichtigung von rückführbaren Maleinsäure- und Bernsteinsäureestern 97 %.

## Patentansprüche

1. Verfahren zur Herstellung von 1,4-Butandiol und/oder Tetrahydrofuran durch katalytische Hydrierung von Verbindungen der allgemeinen Formel

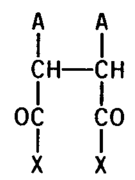

in der A Wasserstoff oder beide A zusammen eine Einfachbindung, X eine HO- oder RO-Gruppe, R einen Alkylrest mit 1 bis 6 C-Atomen, oder beide X zusammen das Ringglied -O- bedeuten, bei Temperaturen von 100 bis 350 °C und Drücken von 50 bis 350 bar, dadurch gekennzeichnet, daß man einen Katalysator verwendet, der Cobalt und mindestens eines der Elemente Kupfer und Phosphor und ggf. noch eines oder mehrere der Elemente Mangan, Molybdän und Natrium enthält.

2. Verfahren nach Anspruch 1, dadurch gekennzeichnet, daß man die Hydrierung in Gegenwart eines aliphatischen Alkohols und eines Katalysators vornimmt, der Cobalt und mindestens eines der Elemente Kupfer, Phosphor oder Mangan enthält.

3. Verfahren nach Anspruch 1, dadurch gekennzeichnet, daß man einen Katalysator verwendet, der neben Cobalt mindestens zwei der Elemente Kupfer, Phosphor und Molybdän enthält.

4. Verfahren nach Anspruch 1, dadurch gekennzeichnet, daß man einen Katalysator verwendet, der Cobalt und mindestens zwei der Elemente Mangan, Kupfer, Phosphor und Molybdän enthält.

5. Verfahren nach Anspruch 1, dadurch gekennzeichnet, daß man einen Katalysator verwendet, der neben Cobalt mindestens drei der Elemente Kupfer, Phosphor, Molybdän und Mangan enthält.

6. Verfahren nach Anspruch 1, dadurch gekennzeichnet, daß man einen Katalysator verwendet, der Cobalt und mindestens drei der Elemente Mangan, Kupfer, Phosphor, Molybdän und Natrium enthält.

7. Verfahren nach Anspruch 1, dadurch gekennzeichnet, daß die aktive Masse des Katalysators mindestens zu 40 Gew.% aus Cobalt besteht.

8. Verfahren nach Anspruch 1, dadurch gekennzeichnet, daß man einen Katalysator verwendet, dessen aktive Masse mindestens zu 40 Gew.% aus Cobalt besteht und als weitere aktive Bestandteile bis zu 10 Gew.% Mangan, bis zu 20 Gew.% Phosphorsäure und bis zu 1 Gew.% Natrium enthält.

**9.** Verfahren nach Anspruch 1, dadurch gekennzeichnet, daß man einen Katalysator verwendet, dessen aktive Masse mindestens zu 40 Gew.% aus Cobalt besteht und als weitere aktive Bestandteile bis zu 10 Gew.% Mangan, bis zu 30 Gew.% Kupfer, bis zu 5 Gew.% Molybdän, bis zu 20 Gew.% Phosphorsäure und bis zu 1 Gew.% Natrium enthält.

**10.** Verfahren nach Anspruch 1, dadurch gekennzeichnet, daß man einen Katalysator verwendet, dessen aktive Masse zu 40 bis 60 Gew.% aus Cobalt, 13 bis 17 Gew.% aus Kupfer 0,5 bis 5 Gew.% aus Molybdän (berechnet als $MoO_3$), 0 bis 8 Gew.% aus Mangan und 0,1 bis 5 Gew,% aus Phosphor (berechnet als $H_3PO_4$) besteht.

## Claims

**1.** A process for the preparation of 1,4-butanediol and/or tetrahydrofuran by catalytic hydrogenation of a compound of the formula

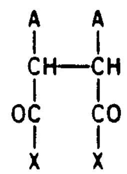

where A is hydrogen or the two radicals A together are a single bond, X is HO or RO, R is alkyl having 1 to 6 carbon atoms, or the two X radicals together are the ring member -O-, at from 100 to 350 °C and at from 50 to 350 bar, which comprises using a catalyst which contains cobalt and at least one of the elements copper and phosphorus and also, if desired, one or more of the elements manganese, molybdenum and sodium.

**2.** A process as claimed in claim 1, wherein the hydrogenation is carried out in the presence of an aliphatic alcohol and in the presence of a catalyst containing cobalt and at least one of the elements copper, phosphorus and manganese.

**3.** A process as claimed in claim 1, wherein a catalyst is used which, in addition to cobalt, contains at least two of the elements copper, phosphorus and molybdenum.

**4.** A process as claimed in claim 1, wherein a catalyst is used which contains cobalt and at least two of the elements manganese, copper, phosphorus and molybdenum.

**5.** A process as claimed in claim 1, wherein a catalyst is used which, in addition to cobalt, contains at least three of the elements copper, phosphorus, molybdenum and manganese.

**6.** A process as claimed in claim 1, wherein a catalyst is used which contains cobalt and at least three of the elements manganese, copper, phosphorus, molybdenum and sodium.

**7.** A process as claimed in claim 1, wherein the active material of the catalyst comprises at least 40% by weight of cobalt.

**8.** A process as claimed in claim 1, wherein a catalyst is used whose active material comprises at least 40% by weight of cobalt and contains, as further active constituents, up to 10% by weight of manganese, up to 20% by weight of phosphoric acid and up to 1% by weight of sodium.

**9.** A process as claimed in claim 1, wherein a catalyst is used whose active material comprises at least 40% by weight of cobalt and contains, as further active constituents, up to 10% by weight of manganese, up to 30% by weight of copper, up to 5% by weight of molybdenum, up to 20% by weight of phosphoric acid and up to 1% by weight of sodium.

10. A process as claimed in claim 1, wherein a catalyst is used whose active material comprises from 40 to 60% by weight of cobalt, from 13 to 17% by weight of copper, from 0.5 to 5% by weight of molybdenum (calculated as $MoO_3$), from 0 to 8% by weight of manganese and from 0.1 to 5% by weight of phosphorus (calculated as $H_3PO_4$).

**Revendications**

1. Procédé de préparation de 1,4-butanediol et/ou de tétrahydrofuranne par hydrogénation catalytique de composés de formule générale

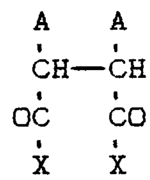

dans laquelle A représente un atome d'hydrogène ou les deux A forment ensemble une liaison simple, X représente un groupement HO ou RO, R étant un reste alkyle à 1-6 atomes de carbone, ou les deux X forment ensemble le chaînon -O-, à des températures de 100 à 350°C et sous des pressions de 50 à 350 bar, caractérisé en ce qu'on utilise un catalyseur qui contient du cobalt, au moins l'un des éléments cuivre et phosphore et éventuellement l'un ou plusieurs des éléments manganèse molybdène et sodium.

2. Procédé selon la revendication 1, caractérisé en ce qu'on conduit l'hydrogénation en présence d'un alcool aliphatique et d'un catalyseur qui contient du cobalt et l'un au moins des éléments cuivre, phosphore et manganèse.

3. Procédé selon la revendication 1, caractérisé en ce qu'on utilise un catalyseur qui contient, outre du cobalt, au moins deux des éléments cuivre, phosphore et molybdène.

4. Procédé selon la revendication 1, caractérisé en ce qu'on utilise un catalyseur qui contient du cobalt et au moins deux des éléments manganèse, cuivre, phosphore et molybdène.

5. Procédé selon la revendication 1, caractérisé en ce qu'on utilise un catalyseur qui contient, outre du cobalt, au moins trois des éléments cuivre, phosphore, molybdène et manganèse.

6. Procédé selon la revendication 1, caractérisé en ce qu'on utilise un catalyseur qui contient du cobalt et au moins trois des éléments manganèse, cuivre, phosphore, molybdène et sodium.

7. Procédé selon la revendication 1, caractérisé en ce que la masse active du catalyseur est composée de cobalt pour au moins 40% en poids.

8. Procédé selon la revendication 1, caractérisé en ce qu'on utilise un catalyseur dont la masse active se compose de cobalt pour au moins 40% en poids et contient, comme autres constituants actifs, jusqu'à 10% en poids de manganèse, jusqu'à 20% en poids d'acide phosphorique et jusqu'à 1% en poids de sodium.

9. Procédé selon la revendication 1, caractérisé en ce qu'on utilise un catalyseur dont la masse active se compose de cobalt pour au moins 40% en poids et contient, comme autres constituants actifs, jusqu'à 10% en poids de manganèse jusqu'à 30% en poids de cuivre, jusqu'à 5% en poids de molybdène, jusqu'à 20% en poids d'acide phosphorique et jusqu'à 1% en poids de sodium

10. Procédé selon la revendication 1, caractérisé en ce qu'on utilise un catalyseur dont la masse active se compose pour 40 à 60% en poids de cobalt, pour 13 à 17% en poids de cuivre, pour 0,5 à 5% en poids de molybdène (calculé en tant que $MoO_3$), pour 0 à 8% en poids de manganèse et pour 0,1 à 5% en poids de phosphore (calculé en tant que $H_3PO_4$).

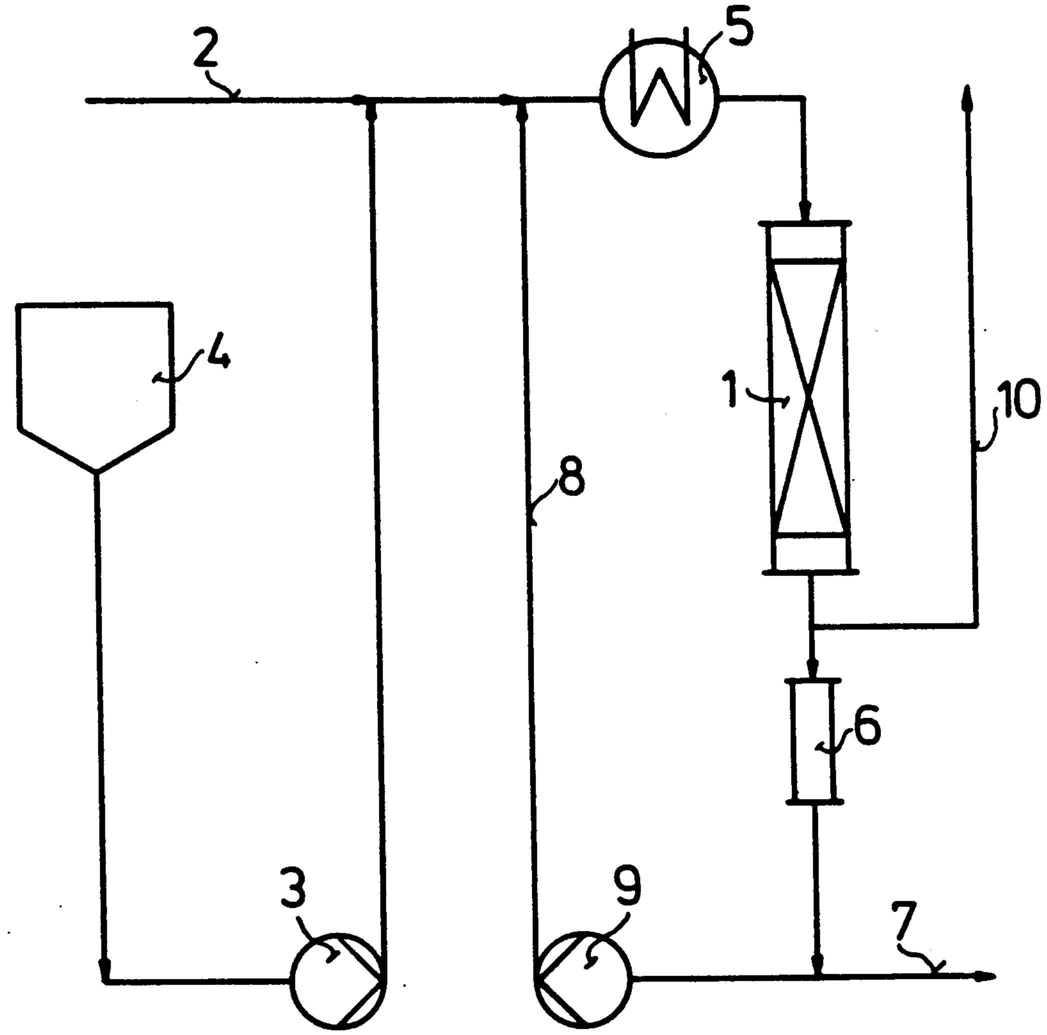
2
5
4
1
10
8
6
3
9
7